# EUROPEAN PATENT APPLICATION

(11) **EP 3 318 171 A1**
(43) Date of publication of application: **09.05.2018**
(21) Application number: 16196820.1
(22) Date of filing: 02.11.2016
(51) Int. Cl.: A61B 1/00, A61B 1/015

(54) **ENDOSCOPIC DEVICE**

(71) Applicant: Siemens Aktiengesellschaft, 80333 München (DE)
(72) Inventor: Andersson, Johan, 61236 Finspang (SE)

(57) **Abstract**

The present invention relates to an endoscopic device (1) comprising a rigid hollow shaft (2) having a first end (2a) and a second end (2b), a scraping device (3) provided at the first end (2a) of the rigid shaft (2), an image transmission device (5) transmitting image signals from the first end (2a) of the rigid shaft (2), a lighting device (6) positioned to illuminate an area next to the first end (2a) of the rigid shaft (2) and a suction device (7) having a suction line (20), which is directly or indirectly connectable to the second end (2b) of the rigid shaft (2) in order to suck material scraped by the scraping device (3). Moreover, the present invention relates to a method for analyzing material present on an outer surface of a turbine component arranged within a turbine housing (102) having at least one manhole (106) and/or at least one inspection port (105) using such endoscopic device (1).

## Description

The present invention relates to an endoscopic device comprising at least one rigid hollow shaft having a first end and a second end, an image transmission device transmitting image signals from the first end of the rigid shaft and a lighting device positioned to illuminate an area next to the first end of the rigid shaft. Furthermore, the present invention relates to a method for analyzing material present on an outer surface of a turbine component arranged within a turbine housing having at least one manhole and/or at least one inspection port, comprising the steps of a) visually inspecting the turbine component, b) scraping off material from the surface of said turbine component, c) collecting the scraped off material and d) analyzing the collected material.

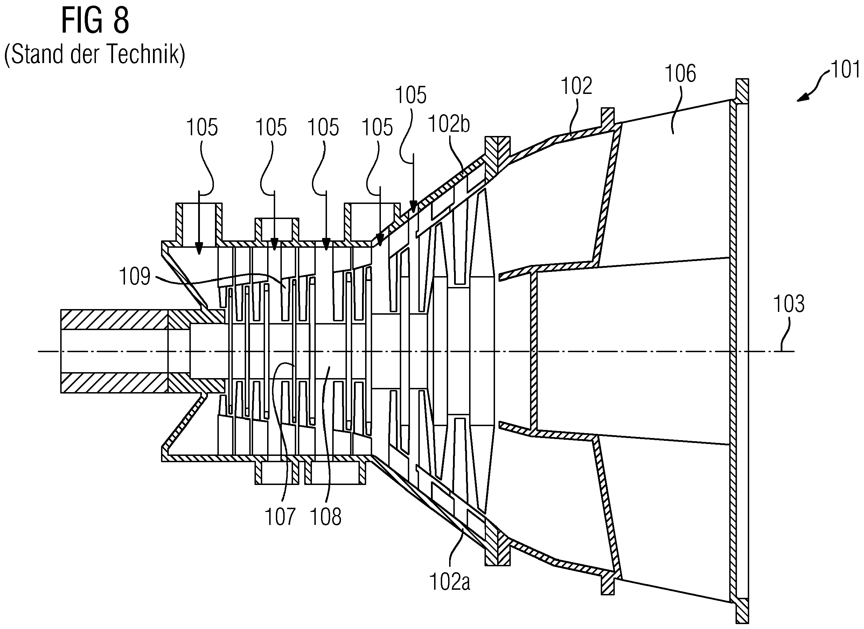

Figure 8 shows a turbine 101 comprising a housing 102. The housing 102 is subdivided into a lower housing half 102a and an upper housing half 102b, which are fixed to each other along a parting line 103. The upper housing half 102b is provided with several inspection ports 105 and a manhole 106 located downstream with respect to the inspection ports 105. Moreover, the turbine 101 comprises a plurality of rotating blades 107, which are fixed to a turbine shaft 108 extending through the housing 102, and stationary vanes 109 fixed to the housing 102, wherein the blades 107 and the vanes 109 together define a total of ten turbine stages in the present case.

In operation hot steam heated by a combustion process is introduced into the housing 102 and led through the turbine stages, where the steam expands and sets the blades 107 and consequently the turbine shaft 108 in motion.

Particularly the development of modern, high-efficiency turbines 101 as shown in figure 8 has led to an increase in deposition, erosion, and corrosion problems. Although several factors influence the formation of deposits on turbine components, the general effect is the same no matter what the cause. Adherent deposits form in the steam passage, distorting the original shape of turbine nozzles and blades. These deposits, often rough or uneven at the surface, increase resistance to the flow of steam. Distortion of steam passages alters steam velocities and pressure drops, reducing the capacity and efficiency of the turbine. Where conditions are severe, deposits can cause excessive rotor thrust. Uneven deposition can unbalance the turbine rotor, causing vibration problems.

Therefore it is necessary to visually inspect the outer surfaces of the concerned turbine components and/or to analyze the composition of material, in particular deposit material, present on these surfaces in regular service intervals in order to assert, whether or not a removal of such material or even a replacement of the corresponding turbine component is necessary. For this purpose the upper half 102b of the housing 102 has to be removed in order to gain access to the concerned turbine components, such as the blades 107 and the vanes 109. However, the opening of the housing 102 is very cumbersome, time-consuming and expensive.

Against this background it is an object of the present invention to provide an alternative method of the above-mentioned kind for analyzing material present on an outer surface of a turbine component arranged within a turbine housing.

In order to solve this object the present invention provides an endoscopic device of the above-mentioned kind, which is characterized in that it further comprises a scraping device provided at the first end of the rigid shaft and a suction device having a suction line, which is directly or indirectly connectable to the second end of the rigid shaft in order to suck material scraped by the scraping device.

By using an endoscopic device according to the invention it is possible to visually inspect a turbine component and/or to scrape off and collect material from the surface of a turbine component from a position outside the turbine without opening the turbine housing by introducing the rigid shaft through or an inspection port or a manhole of the turbine housing. Accordingly, a visual inspection of surfaces of turbine components as well as an analysis of of material present on such surfaces can be performed in a quick and cost efficient manner.

Preferably the rigid shaft has at least one bending in order to enable to reach a specific position within the turbine housing.

The form of the scraping device can be adjusted to enable different applications. Preferably, the scraping device has a spoon-like or speculate form in order to provide a shielding to achieve a high suction power at the first end of the rigid shaft and/or a collector for catching scraped off material.

According to an aspect of the present invention the rigid shaft and the scraping device are formed integrally, i.e. as one piece, in order to provide a good robustness.

Preferably the width of the scraping device essentially corresponds to the diameter of the first end of the rigid shaft. Thus, the scraping device may be manufactured from a conventional shaft by shaping its free end.

The scraping device can also be produced separately and then welded to the rigid shaft in order to provide a larger freedom of design for the scraping device or to manufacture the scraping device from a material different from that of the rigid shaft.

An edge of the scraping device may be tapered towards its free end like a blade in order to facilitate scraping off material from the surface of a turbine component.

Preferably, the rigid shaft and/or the scraping device are made of metal. In this manner the reliability of the endoscopic device is enhanced.

The image transmission device can be provided in the form of a camera system, in particular by using digital technologies as in the case of a video endoscope. Alternatively the image transmission device can be provided by a lens arrangement with an eyepiece positioned at the second end of the rigid shaft.

The image transmission device can comprise a display for displaying signals transmitted by the image transmission device.

According to one aspect of the present invention the suction device comprises an ejector, which is connected to the suction line and a pressure line. The ejector comprises a pipe section with a constricted part. In the constricted part the high pressure of the pressure line is converted into a high velocity, which creates a low pressure at that point, which is used for the suction line. The size of the passage area in the constricted part depends on the suction power needed.

The suction device can comprise a material separator and/or a filter element for separating scraped off material, which is preferably installed in the suction line.

The image transmission device and/or the lighting device can be at least partly arranged within the rigid shaft, wherein the endoscopic device preferably comprises a handling unit receiving the second end of the rigid shaft. Such a handling unit facilitates the handling of the endoscopic device for the user. The handing unit may comprise a grip portion, actuation elements and the-like and is preferably made of plastic.

Alternatively, the image transmission device and/or the lighting device is/are at least partly arranged within a flexible hollow shaft, to which the rigid shaft is detachably fixed, e.g. by means of cable straps, wherein the endoscopic device preferably comprises a handling unit receiving one end of the flexible shaft. Such a handling unit facilitates the handling of the endoscopic device for the user. The handing unit may comprise a grip portion, actuation elements and the-like and is preferably made of plastic.

The endoscopic device preferably comprises a plurality of hollow rigid shafts selectively fixable to the flexible shaft in a detacheable manner.

In order to solve the above-mentioned object the present invention further provides a method of the above mentioned kind, which is characterized in that the material is scraped off from the surface of said turbine component in step b) by means of an endoscopic device having a rigid hollow shaft, in particular an endoscopic device according to the present invention, wherein the rigid shaft is introduced in the turbine housing through said at least one manhole or said at least one inspection port.

Preferably, the rigid shaft is bended on-site prior to performing step b) to enable to reach a specific position within the turbine housing during step b). Alternatively, a plurality of rigid shafts having different bends can be provided and a suitable rigid shaft can be chosen in order to perform step b).

Preferably the scraped off material is collected in step c) using a suction device, which is particularly connected to a material separator and/or a filter element. Thereby it is possible to collect the scraped off material quickly and reliably.

Further features and aspects of the present invention will become apparent to a skilled person from the following description of an embodiment of an endoscopic device according to the present invention with reference to the accompanying drawing. In the drawing
- Figure 1: is a schematic view of an endoscopic device according to a first embodiment of the present invention;

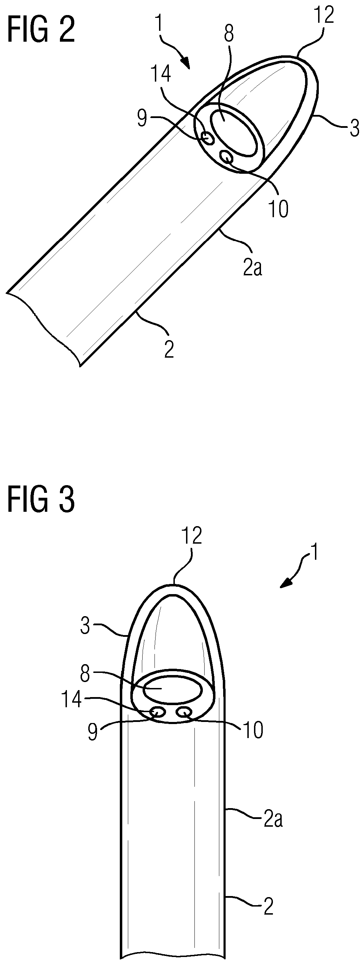

- Figure 2: is a side view of a first end of a rigid shaft of the endoscopic device shown in figure 1;
- Figure 3: is a top view of the first end of the rigid shaft shown in figure 2;
- Figure 4: is a cross-sectional view of a turbine with the endoscopic device inserted through an inspection port;
- Figure 5: is a schematic view of an endoscopic device according to a second embodiment of the present invention;
- Figure 6: is a side view of a first end of a rigid shaft attached to a flexible shaft of the endoscopic device shown in figure 5;
- Figure 7: is a top view of the first end of the arrangement shown in figure 6; and
- Figure 8: is a cross-sectional view of a turbine according to the state of the art.

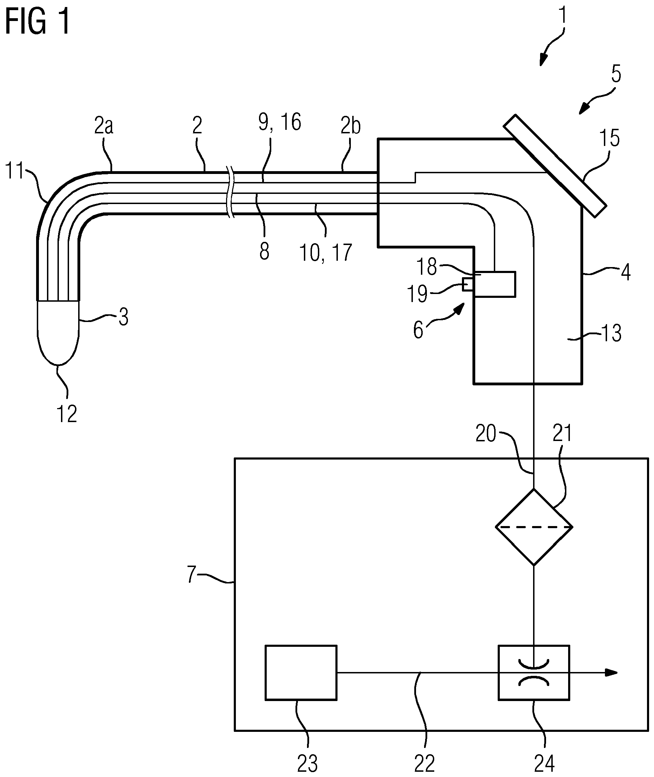

Figure 1 shows an endoscopic device 1 according to a first embodiment of the present invention. The endoscopic device 1 comprises as main components a rigid hollow shaft 2, a scraping device 3, a handling unit 4, an image transmission device 5, a lighting device 6 and a suction device 7.

The rigid hollow shaft 2 has a first end 2a and a second end 2b. It defines three channels extending therethrough, namely a suction channel 8, a first receiving channel 9 and a second receiving channel 10, wherein the suction channel 8 presently takes up at least 50% of the cross section area of the rigid hollow shaft 2. The rigid shaft 2 has one bending 11 and is made of metal. However, it should be noted that the number of bendings 11 and/or the material of the rigid shaft 2 may vary.

The scraping device 3 is provided at the first end 2a of the rigid shaft 2. In the present case the scraping device 3 has a spoon-like form having an outer edge 12 tapered towards its free end like a blade. However, the scraping device 3 may also have another form, such as a speculate form to name an example. The scraping device 3 is made of metal. It can be welded or brazed to the first end of the rigid shaft 2 or can be integrally formed therewith from one single piece. In the first case the rigid shaft 2 and the scraping device can comprise different material compositions. For example, the scraping device 3 can be made from a more wear resistant material than the rigid shaft 2.

The handling unit 4 receives the second end 2b of the rigid shaft 2. In the present case the handling unit comprises a grip portion 13 and is made of plastic.

The image transmission device 5 is presently provided in the form of a camera system and comprises a CCD chip 14 at the first free end 2a of the rigid shaft 2, a display 15 positioned at the handling unit 4 and an image signal transmission cable 16 arranged within the first receiving channel 9 of the rigid shaft 2 and connecting the CCD chip 14 to the display 15. Although a digital image transmission device 5 in the form of a camera system is preferred, other technical implementations are possible, such as a lens arrangement with an eyepiece arranged at the handling unit 4 or the like.

The lighting device 6 comprises a glass fiber bundle 17 extending through the second receiving channel 10 of the rigid shaft 2 and a light source 18 connected therewith and arranged within the handling unit 4. The light source 18 may be provided as a LED and can be manipulated by means of a light switch 19 positioned at the grip portion 13 of the handling unit 4.

The suction device 7 presently comprises a suction line 20 connected to the suction channel 8 of the rigid shaft 2, a filter element or material separator 21 integrated in the suction line 20, a pressure line 22 connectable to a high pressure reservoir 23 and an ejector 24 being connected to the suction line 20 as well as to the pressure line 22. However it should be noted, that a vacuum pump connected to the suction line 20 may replace the pressure line 22, the high pressure reservoir 23 and the ejector 24 in order to create an underpressure within the suction line 20.

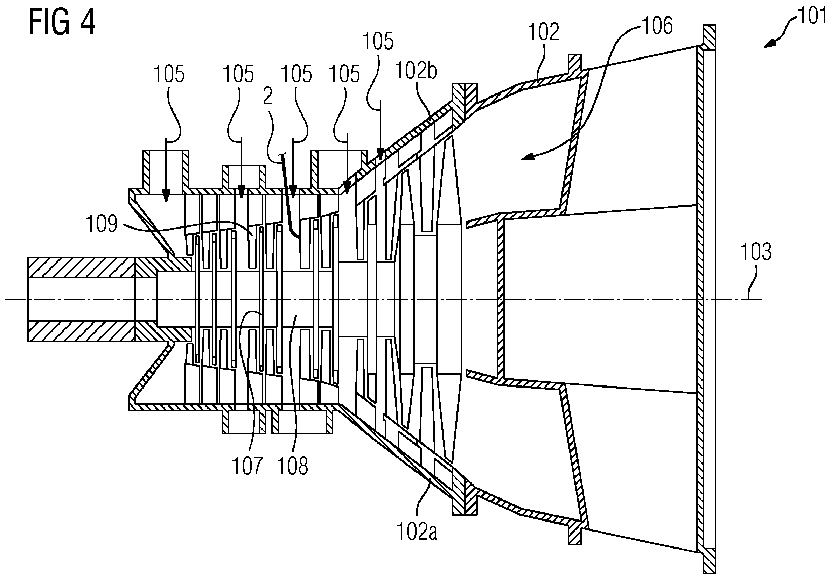

For inspecting a turbine component arranged within a turbine housing 102, such as a blade 107 or a vane 109, and/or for analyzing material present on an outer surface of said turbine component, the rigid shaft 2 of the endoscopic device 1 is introduced into the housing 102 through an inspection port 105, as shown in figure 4, or through the manhole 106 and is moved forward to the turbine component to be checked. The image transmission device 5, the lighting device 6 and the bending 11 of the rigid shaft 2 thereby help guiding the rigid shaft 2 through the inside of the housing 102 in order to prevent collisions with obstacles on the way from the inspection port 105 to the turbine component. The image transmission device 5 enables the operator to observe the area around the first free end 2a of the rigid shaft 2 on the display 15 by transmitting the image signals of the CCD chip 14 through the image transition cable 16 to a processor of the display 15. The lighting device 6 illuminates an area next to the first end 2a of the rigid shaft 2 by leading the light emitted from the light source 18 through the light fiber bundle 17. The bending 11 is formed in a manner to pass a known obstacle. As soon as the component to be inspected is reached, the operator can manually scrape material from its surface by means of the scraping device 3. Scraped off material may be first collected by the spoon-like scraping device 3 and is then sucked through the suction channel 8 and the suction line 20 to the filter element or material separator 21, where it is stored. The necessary underpressure within the suction line 20 is created by compressed air created within the high pressure reservoir 23 and guided through the pressure line 22 to the ejector 24. Thereafter, the scraped off material collected within the filter element or material separator 21 can be removed for further analyzation.

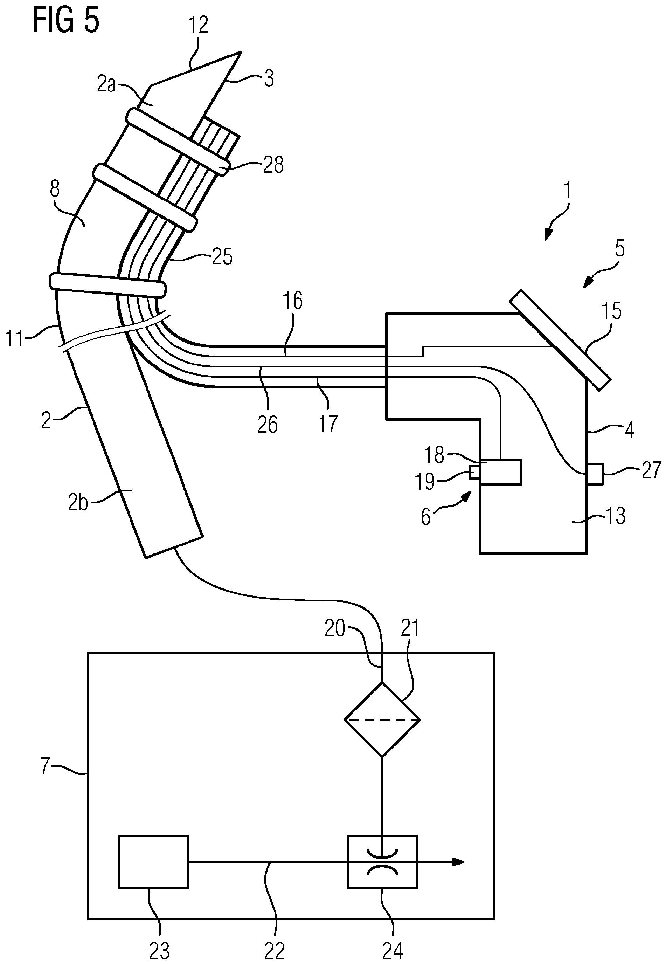

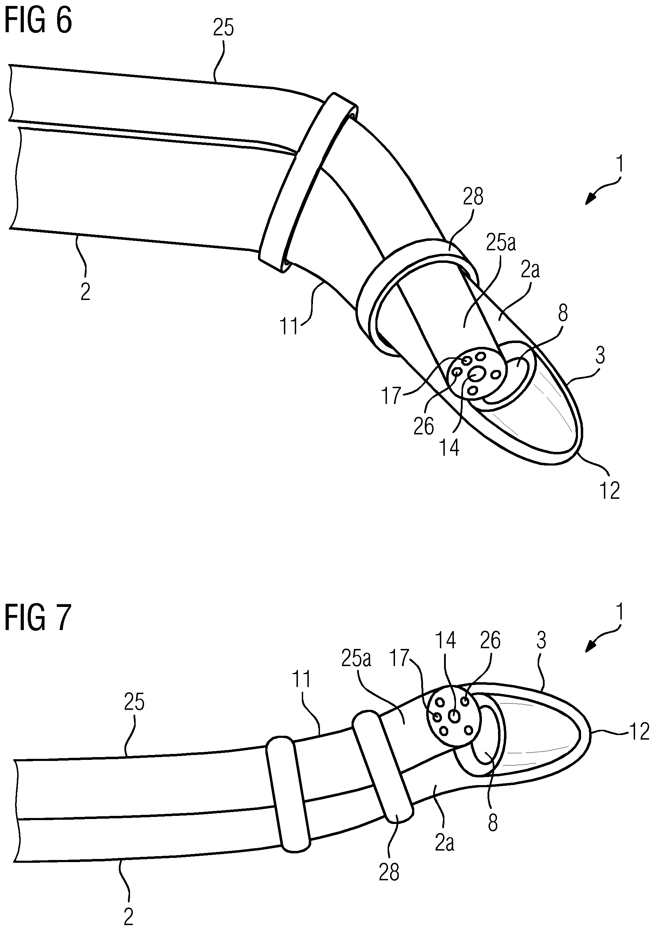

Figures 5 to 7 shows an endoscopic device 1 according to a second embodiment of the present invention, which has a similar structure as the above-described endoscopic device 1 according to the first embodiment, for which reason same reference numbers are used to denote same or similar components.

In contrast to the endoscopic device 1 described before, the rigid hollow shaft 2 of the endoscopic device 1 according to the second embodiment is provided as a separate single component, which only defines the suction channel 8. Moreover, a flexible shaft 25 is provided having its one end connected to the handling unit 4, its free end provided with the CCD chip 14 and receiving the image signal transmission cable 16, the glass fiber bundle 17 and a plurality of Bowden cables 26 in corrsponding receiving channels, wherein the Bowden cables 26 are manipulateable by an operator via a joystick control 27 arranged at the handling unit 4 in order to deform or bend the flexible shaft 25. Consequently, the flexible shaft 25 and the handling unit 4 together define a video boroscope as know in the art. The rigid shaft 2 is detachably fixed at least to the free end of the flexible shaft 25 via cable ties 28.

In order to check a turbine component arranged within the turbine housing 102, such as a blade 107 or a vane 109, first the flexible shaft 25 not having the rigid shaft 2 fixed thereto, is introduced in the turbine housing 102 via an inspection port 105 or the manhole 106 and led to the turbine component to be checked. If negative changes on the surface of said turbine component, e.g. deposits, are located during this visual inspection, the flexible shaft 25 is pulled out of the turbine housing 102 again. Then, a rigid shaft 2 having a suitable bending or suitable bendings 11, which can be on-site, is fixed to the flexible shaft 25. Subsequently the rigid shaft 2 and the flexible shaft 25 are together introduced into the turbine housing 102 via an inspection port 105 or the manhole 106 and guided to the turbine component inspected before in order to scrape off and collect sample material from its surface as described before.

It should be noted that the above-described embodiment serves only as an example, and that modifications are possible without leaving the scope of protection defined by the accompanying claims. In particular the shape of the rigid shaft 2, such as its length as well as the number and location of bendings 11, the shape of the scraping element 3, etc. can be changed in order to adapt these components to a specific intended application.

## Claims

1. Endoscopic device (1) comprising at least one rigid hollow shaft (2) having a first end (2a) and a second end (2b), an image transmission device (5) transmitting image signals from the first end (2a) of the rigid shaft (2) and a lighting device (6) positioned to illuminate an area next to the first end (2a) of the rigid shaft (2), **characterized in that**
the endoscopic device (1) further comprises a scraping device (3) provided at the first end (2a) of the rigid shaft (2) and a suction device (7) having a suction line (20), which is directly or indirectly connectable to the second end (2b) of the rigid shaft (2) in order to suck material scraped by the scraping device (3).

2. Endoscopic device (1) according to claim 1,
**characterized in that**
the rigid shaft (2) has at least one bending (11).

3. Endoscopic device (1) according to any one of the preceding claims,
**characterized in that**
the scraping device (3) has a spoon-like or spatulate form.

4. Endoscopic device (1) according to any one of the preceding claims,
**characterized in that**
the scraping device (3) is integrally formed with the rigid shaft (2).

5. Endoscopic device (1) according to any one of the preceding claims,
**characterized in that**
an edge of the scraping device (3) is tapered towards its free end like a blade.

6. Endoscopic device (1) according to any one of the preceding claims,
**characterized in that**
the rigid shaft (2) and/or the scraping device (3) is/are made of metal.

7. Endoscopic device (1) according to any one of the preceding claims,
**characterized in that**
the image transmission device (5) is provided in the form of a camera system.

8. Endoscopic device (1) according to any one of the preceding claims,
**characterized in that**
the image transmission device (5) comprises a display (15) for displaying signals transmitted by the image transmission device (5), said display (15) is preferably fixed to the handling unit (4), if present.

9. Endoscopic device (1) according to any one of the preceding claims,
**characterized in that**
the suction device (7) comprises an ejector (24),
which is connected to the suction line (20) and a pressure line (22).

10. Endoscopic device (1) according to any one of the preceding claims,
**characterized in that**
the suction device (7) comprises a material separator and/or a filter element (21),
which is preferably installed in the suction line (20).

11. Endoscopic device (1) according to any one of the preceding claims,
**characterized in that**
the image transmission device (5) and/or the lighting device (6) is/are at least partly arranged within the rigid shaft (2),
wherein the endoscopic device (1) preferably comprises a handling unit (4) receiving the second end (2b) of the rigid shaft (2).

12. Endoscopic device (1) according to one of the claims 1 to 10,
**characterized in that**
the image transmission device (5) and/or the lighting device (6) is/are at least partly arranged within a flexible hollow shaft (25),
to which the rigid shaft (2) is detachably fixed, wherein the endoscopic device (1) preferably comprises a handling unit (4) receiving one end of the flexible shaft (25).

13. Endoscopic device (1) according to claim 12,
**characterized in that**
it comprises a plurality of hollow rigid shafts (2) selectively fixable to the flexible shaft (25) in a detachable manner.

14. Method for analyzing material present on an outer surface of a turbine component arranged within a turbine housing (102) having at least one manhole (106) and/or at least one inspection port (105), comprising the steps:
a) visually inspecting the turbine component,
b) scraping off material from the surface of said turbine component,
c) collecting the scraped off material and
d) analyzing the collected material,
wherein the material is scraped off from the surface of said turbine component in step b) by means of an endoscopic device having a rigid hollow shaft (2), in particular an endoscopic device (1) according to any one of the preceding claims,
whose rigid shaft (2) is introduced in the turbine housing (102) through said at least one manhole (106) or said at least one inspection port (105).

15. Method according to claim 14,
**characterized in that**
the the rigid shaft (2) is bended on-site to enable to reach a specific position within the turbine housing (102) during step b) or **in that** a plurality of rigid shafts (2) having different bends is provided and a suitable rigid shaft (2) is chosen in order to perform step b).

16. Method according to claim 14 or 15,
**characterized in that** the scraped off material is collected in step c) using a suction device (7),
which is particularly connected to a material separator and/or a filter element (21).

## Amended claims

### Amended claims in accordance with Rule 137(2) EPC.

1. Endoscopic device (1) comprising at least one rigid hollow shaft (2) having a first end (2a) and a second end (2b), an image transmission device (5) transmitting image signals from the first end (2a) of the rigid shaft (2) and a lighting device (6) positioned to illuminate an area next to the first end (2a) of the rigid shaft (2), wherein the endoscopic device (1) further comprises a scraping device (3) provided at the first end (2a) of the rigid shaft (2) and a suction device (7) having a suction line (20), being directly or indirectly connectable to the second end (2b) of the rigid shaft (2) in order to suck material scraped by the scraping device (3), and wherein the scraping device (3) has a spoon-like or spatulate form.

2. Endoscopic device (1) according to claim 1,
**characterized in that**
the rigid shaft (2) has at least one bending (11).

3. Endoscopic device (1) according to any one of the preceding claims,
**characterized in that**
the scraping device (3) is integrally formed with the rigid shaft (2).

4. Endoscopic device (1) according to any one of the preceding claims,
**characterized in that**
an edge of the scraping device (3) is tapered towards its free end like a blade.

5. Endoscopic device (1) according to any one of the preceding claims,
**characterized in that**
the rigid shaft (2) and/or the scraping device (3) is/are made of metal.

6. Endoscopic device (1) according to any one of the preceding claims,
**characterized in that**
the image transmission device (5) is provided in the form of a camera system.

7. Endoscopic device (1) according to any one of the preceding claims,
**characterized in that**
the image transmission device (5) comprises a display (15) for displaying signals transmitted by the image transmission device (5), said display (15) is preferably fixed to the handling unit (4), if present.

8. Endoscopic device (1) according to any one of the preceding claims,
**characterized in that**
the suction device (7) comprises an ejector (24), which is connected to the suction line (20) and a pressure line (22).

9. Endoscopic device (1) according to any one of the preceding claims,
**characterized in that**
the suction device (7) comprises a material separator and/or a filter element (21),
which is preferably installed in the suction line (20).

10. Endoscopic device (1) according to any one of the preceding claims,
**characterized in that**
the image transmission device (5) and/or the lighting device (6) is/are at least partly arranged within the rigid shaft (2),
wherein the endoscopic device (1) preferably comprises a handling unit (4) receiving the second end (2b) of the rigid shaft (2).

11. Endoscopic device (1) according to one of the claims 1 to 9,
**characterized in that**
the image transmission device (5) and/or the lighting device (6) is/are at least partly arranged within a flexible hollow shaft (25),
to which the rigid shaft (2) is detachably fixed, wherein the endoscopic device (1) preferably comprises a handling unit (4) receiving one end of the flexible shaft (25) .

12. Endoscopic device (1) according to claim 12,
**characterized in that**
it comprises a plurality of hollow rigid shafts (2) selectively fixable to the flexible shaft (25) in a detachable manner.

13. Method for analyzing material present on an outer surface of a turbine component arranged within a turbine housing (102) having at least one manhole (106) and/or at least one inspection port (105), comprising the steps:
a) visually inspecting the turbine component,
b) scraping off material from the surface of said turbine component,
c) collecting the scraped off material and
d) analyzing the collected material,
wherein the material is scraped off from the surface of said turbine component in step b) by means of an endoscopic device according to any one of the preceding claims,
whose rigid shaft (2) is introduced in the turbine housing (102) through said at least one manhole (106) or said at least one inspection port (105).

14. Method according to claim 13,
**characterized in that**
the the rigid shaft (2) is bended on-site to enable to reach a specific position within the turbine housing (102) during step b) or **in that** a plurality of rigid shafts (2) having different bends is provided and a suitable rigid shaft (2) is chosen in order to perform step b).

15. Method according to claim 13 or 14,
**characterized in that** the scraped off material is collected in step c) using a suction device (7),
which is particularly connected to a material separator and/or a filter element (21).
